Europäisches Patentamt

European Patent Office

Office européen des brevets

(11) Publication number: **0 087 899**

**B1**

# (12) EUROPEAN PATENT SPECIFICATION

(45) Date of publication of patent specification: **30.12.86**

(21) Application number: **83300862.6**

(22) Date of filing: **18.02.83**

(51) Int. Cl.⁴: **B 01 L 3/00, G 01 N 33/543**

(54) **Multi-well screening assembly for immunoassay procedures.**

(30) Priority: **03.03.82 US 354554**

(43) Date of publication of application:
**07.09.83 Bulletin 83/36**

(45) Publication of the grant of the patent:
**30.12.86 Bulletin 86/52**

(84) Designated Contracting States:
**BE DE FR GB IT NL SE**

(56) References cited:
**EP-A-0 042 755**
**FR-A-2 277 563**
**FR-A-2 362 397**
**GB-A-2 079 181**
**US-A-4 111 754**
**US-A-4 200 613**

(73) Proprietor: **Becton, Dickinson and Company**
**Mack Centre Drive P.O. Box 2224**
**Paramus New Jersey 07652-1149 (US)**

(72) Inventor: **Sapatino, Bruno V.**
**1401 S. Port Dr.**
**Oxnard, CA 93030 (US)**
Inventor: **Johnson, Luther R.**
**878 Capitan Street**
**Thousant Oaks, Ca 91320 (US)**

(74) Representative: **Ruffles, Graham Keith et al**
**MARKS & CLERK 57-60 Lincoln's Inn Fields**
**London WC2A 3LS (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

The present invention relates to a multi-well screening system useful in immunoassay procedures, and more particularly, concerns such a screening assembly suitable for the screening of hybrid cell cultures such as hybridomas. The present invention also relates to a carrier plate useful in immunoassay procedures and having elements thereof adapted to absorb immunogenic substances thereto.

Monoclonal antibodies are being investigated by researchers for a variety of uses, including the treatment of cancer. It is now well known that monoclonal antibodies can be produced in large quantities from hybridomas. A hybridoma is a hybrid cell which has been produced by joining together a cancer cell with an antibody-making cell. Inasmuch as these hybridomas can produce specific antibodies in large quantities, it is desirable to be able to detect and quantify the presence of the specific hybridoma under investigation.

The search for a certain hybridoma can be very tedious and time-consuming. In the research laboratory, the quest for a certain antibody may start with the injection of an antigen, against which the certain antibody will form, into a mouse. Following removal of the mouse's spleen, the spleen cells are fused with cancer cells. It is to be appreciated that after this process of fusing cancer cells and spleen cells, the researcher must be able to detect which of these fused cells are actually producing the desired antibodies. Only a very small percentage of the cells which the researcher started with will actually fuse together to form a hybrid. This search for fused cells usually includes the distribution of the combined spleen cells and cancer cells into various compartments in order to make the proper identification. Eventually, the researcher attempts to isolate not only the hybridomas, but also to isolate those hybridomas which are producing the sought for antibody. Once again, the researcher may rely upon various compartmentalized trays or tubes in order to complete this isolation.

In the detection and quantification of the various proteins, such as monoclonal antibodies produced by hybridomas, the researcher may rely upon one or more assays. For example, he may use the enzyme-linked immunosorbent assay (ELISA) or the radioimmunoassay (RIA). These immunoassays are very well known to researchers in this field. Both of these assays may utilize test equipment including multiple compartments or wells so that processing of each may be performed simultaneously on a microtiter basis. For example, Schlieicher and Schuell, Inc, Keene, New Hampshire 03431, USA, utilize a 96 place microsample filtration manifold which has direct application in the DNA/RNA hybridization research field. Also, Klebe, Robert J, et al, in "Replica Plating of Cultured Mammalian Cells," *Somatic Cell Genetics*, Vol 7, No. 3, 1981, pp 271—180, describe a device which permits the replica plating of lymphocytes as well as substrate adherent fibroblasts. In this study, the authors relied on ELISA assays with 96 well microtiter plates.

Beads have been utilized in these assay procedures in order to link antigen or antibody thereto in the detection of the sought for biological substance. For example, Litton Bionetics Laboratory Products, Kensington, Maryland, USA, have described the use of a "BIO-BEAD"® which is used for the rapid screening of serum specimens for the presence of detectable antibodies to the TORCH group of antigens. These beads include a magnetic component for use in the Litton Bionetics' system. Micro-Media Systems, Inc, Potomac, Maryland, USA, have disclosed an MIC and bacterial identification device which includes a tray with a multiplicity of wells and a cover with downwardly depending pins which fit in alignment in the wells. These pins are adapted to permit protein (antigen or antibody) to become adsorbed to the surface thereof. It has been found, however, that elongate pins may cause a capilliary action when dipped into fluid in the wells. As a result, accuracy of fluid transferred to the pin may be compromised. Furthermore, fluid sometimes drips from these pins after the cover is removed from the tray with the wells. This may result in cross-contamination of the fluid into adjacent wells.

European Pktent Specification 42755A describes a specific binding assay, for example an immunoassay, which utilizes a microtitre vessel containing a round-section, round-bottom displacer body of slightly smaller and complimentary shape. The adaption of the displacer body results in a high surface-to-volume ratio for liquid in the well and thus more efficient contact with reactants coated on the surface of the displacer body.

UK Patent Specification 2079181A describes a support means for holding and identifying a plurality of spheres, comprising a plate with an array of holes and accessible through each hole a cage-like structure of helical or basket form. The aim is to provide maximum access of a solution to the spheres on immersion, and optimum draining on removal of the spheres from the solution.

French Patent Specification 2362397 discloses apparatus comprising a microtitre plate with hollows for receiving samples for analysis and solid supports which can be loaded with reactants, the apparatus being characterized by supports in the form of thin slabs or small rods fixed to an upstanding handle and which can be introduced in to the hollows of the microtitre plate. The apparatus is for use in a radioimmunoassay, and in the illustrated embodiment the thin slabs are attached to the handle by rupturable narrow necks.

US Patent Specification 4200613 describes radioimmunoassay apparatus which comprises a bead for carrying a dry antibody and a gripper for gripping the bead, the gripper comprising an elongate handle and a clasp portion. The heads and grippers are typically assembled as rows on carriers, and in a preferred apparatus the letters "L" and "R", corresponding to "left" and "right" are embossed or printed or otherwise affixed to each carrier. In this way, the user is usually warned of the correct orientation for each row.

While the above-mentioned devices may be known and used, improvements are being sought in the multiple-well approach to screening of biological substances, such as hybridomas. It is to such improvements that the present invention is directed.

The present invention provides a carrier plate which comprises:

a generally rectangular support body,

a plurality of posts arranged in a plurality of rows and extending from a face of the body with each post having a substantially spherical bead at its distal end adapted to bind an immunogenic substance to its surface, each substantially spherical bead being integral with the respective post and not being intended to be removable either from the respective post or generally from the support body,

a generally rectangular wall extending from the support body to surround the plurality of posts, the wall having two shorter sides and two longer sides, and

the wall being squared off at two corners common to a shorter side as an aid to alignment of the carrier plate with a correspondingly configured tray.

The carrier plate is suited for multi-well screening assembly of the present invention which comprises:

a carrier plate in accordance of the present invention, and a tray arranged in a plurality of rows and a generally rectangular wall extending to surround the plurality of wells, the wall having two shorter sides and two longer sides, and the wall being squared off at two corners common to a shorter side and being dimensioned and configured for uniquely positioning of the carrier plate on the tray with each post extending from the plate to align with a respective one of the wells.

The spherical beads are adapted to bind an immunogenic substance to the surface thereof and, to this end, are preferably made of plastics, with the plastics of choice being styrene acrylonitrile.

In a preferred embodiment of the invention, the tray includes 96 wells arranged in orthogonal columns and rows, with the carrier plate including a corresponding number of posts for alignment with the wells.

In accordance with the present invention, simultaneous processing of all wells of a microtiter for biological substance screening purposes can be achieved. By employing an immunoassay, such as ELISA or the like, the present invention allows the determination of those wells which contain hydridoma cells which produce antibodies. Thus, the present invention further provides an enzyme-linked immunosorbent assay ("ELISA") for screening of hybridoma cultures, characterized in that an assembly of this invention is employed as part of the equipment for the assay, with the hybridoma cultures having been grown in the wells of the tray and the substantially spherical beads being immersed in the cultures, removed and tested in accordance with the ELISA procedure known *per se.*

It is also desirable to utilize the present invention in determining which of the wells of the screening assembly contain hybridoma cells and also contain specific monoclonal antibodies. The present invention allows the handling of all wells, preferably 96, in one step instead of much smaller numbers of wells which are presently being handled by researchers. In addition, in utilizing the beads of the present invention, only an extremely small amount of fluid is withdrawn from the wells upon completion of the individual tests, that is, only the fluid that is retained on the beads is removed from the wells. It is appreciated that the simultaneous transfer of up to 96 protein adsorbing substrate units to various other multi-well or multi-tube devices contributes to saving material and labour in the performance of immunoassay tests. The structure of the present invention desirably eliminates or minimizes cross-contamination among the plurality of wells during the simultaneous transfer of the aseptic fluids. The components of the present invention may also be made inexpensively so as to be disposable after use.

The present invention will now be illustrated by way of non-limiting example with reference to the accompanying drawings, in which:

Fig. 1 is a perspective view illustrating a preferred embodiment of the multi-well screening assembly of the present invention, with the preferred tray and carrier plate separated from each other as they may appear before use;

Fig. 2 is a side elevation, partly in section, illustrating the assembled components of the embodiment of Fig 1; and

Fig. 2a is a view similar to Fig. 2 illustrating an alternative tray element of the present invention.

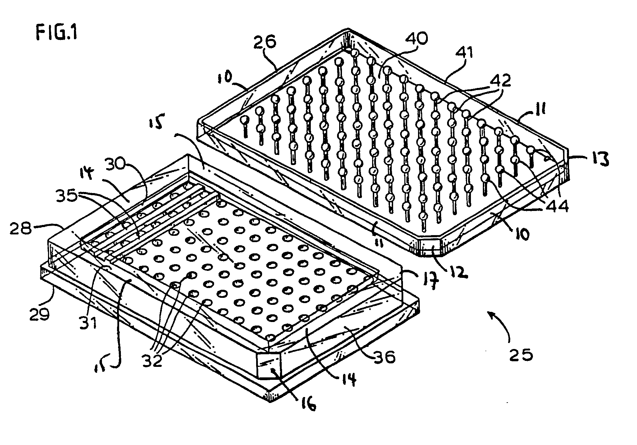

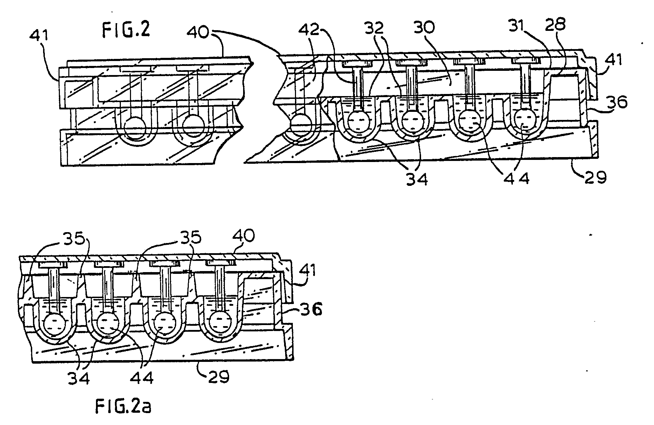

Adverting now to the drawings, and Fig. 1 and 2 in particular, there is illustrated a multi-well screening assembly 25 which is useful in immunoassay procedures. This assembly is comprised of two general components: a carrier plate 26 and a tray 28 onto which the carrier plate is positioned during use, as more clearly illustrated in Fig. 2.

Tray 28 generally has a substantially planar body 29 which may be supported on a flat surface. Formed within body 29 is a deep recess 30 so that a web 31 surrounds the recess. Formed within recess 30 is a plurality of separated, individualized wells 32, which preferably have cylindrically shaped sidewalls and include a spherically shaped bottom 34 inside the tray body so that fluid may be deposited therein. Wells 32 are preferably formed in the tray in an orthogonal array of rows and columns. In the embodiments being described, there are 96 wells in the tray, with 12 rows each with 8 wells. The individualized wells are provided in the tray to minimize the volume of solution required to coat beads 44. The wells may all be placed in the bottom of one large recess 30 as shown in Fig. 2, or each row may be separated with barriers 35 as shown in Fig. 2a, or one or two rows only may be separated as seen in Fig. 1. The advantage of using a single recess is that it requires only the one-time addition of a fixed volume of solution to submerge all wells. The advantage of separated rows is that the assembly allows for use of individual rows for controls; however, it requires the individual addition of solution to each row. The separation of one or two rows only is a compromise which provides both control provisions and reduced filling operations.

While the volume of each well may vary according to many factors, it is preferred, in the embodiment being described, that each well have the capacity to hold approximately one hundred microlitres of fluid.

In order to minimize expense and render the tray disposable, it is preferred that it be made of plastics material. To minimize the amount of solution used, it is also preferred that the material selected for the tray be made of a non-protein adsorbent material such as polypropylene, or polyethylene.

Turning now to carrier plate 26, it is comprised of generally planar support body 40 surrounded by a raised wall 41. The dimensions of wall 41 allow the carrier plate to be positioned on tray 28 so that wall 41 surrounds wall 36 on the tray, as more clearly seen in Fig. 2. Protruding from the planar surface of body 40 is a plurality of posts 42. These posts are arranged in the same pattern as wells 32 in the tray and generally correspond to the same number of wells. In the embodiment being described there are thus 96 posts orthogonally arranged in 12 rows of 8 posts each.

When the carrier plate is positioned onto the tray as illustrated in Fig. 2, each post depends into one of the wells in the tray. It is preferred that the posts all have the same diameter and length so that consistency and uniformity can be maintained. A substantially spherical bead 44 is connected to the distal end of each post so that the bead depends deepest into the well when the carrier plate is positioned on the tray. In order to allow the beads to enter the wells, it is preferred that they have a diameter of about 3.2 millimeters, although this size may vary according to circumstances. In the preferred embodiment of the present invention, carrier plate 26 is preferably an integrally formed or moulded structure. Plastics are the materials of choice, such as styrene acrylonitrile, polypropylene, polyethylene and, polystyrene. However, irrespective of the material of carrier plate 26 in general, beads 44 must be from materials which will allow proteins to be absorbed on the surfaces thereof. As used herein, the term proteins includes any immunogenic substance, be it antigen or antibody. The bead material should be capable of establishing a hydrophobic bond with the protein so that the protein can be strongly adsorbed on the surface of the bead material. It has been found that plastic materials satisfy this criteria of adsorbing protein from a properly prepared fluid medium which contacts the beads. The preferred bead material for purposes of the present invention is styrene acrylonitrile, although other plastic materials may be employed, such as nylon, polystyrene and the like.

While the invention as described herein can be used by researchers for the detection and quantification through the immunoassay method, of many different biological substances, it finds significant application in the testing for monoclonal antibodies and, therefore, for specific hybridoma cells. Specifically, a simple test may be performed using the present invention to make a determination in which of the wells there are antibody-producing hybridoma cells. Such a determination is possible due to the interaction between antibody and antigen and the manner of protein treatment of the beads, disks or other bodies which adsorb immunogenic substances thereto. A more complete screening test utilizing the present invention can determine which of the wells that contain hybridoma cells also contain specific monoclonal antibody-producing hybridoma cells.

An example of the aforementioned simple test, to which the present invention, of course, is not limited, is as follows: a carrier plate which includes beads, disks or other bodies thereon capable of adsorbing proteins, is positioned on a tray preferably containing 96 wells each containing hybridoma cells covered by HAT (containing Hypoxanthine, Aminopterin, Thymidine) fluid medium. If beads are utilized on the carrier plate, the medium then bathes the beads. This step may last between 15 and 45 minutes and is usually carried out at room temperature and under aseptic conditions. Following this bathing step, the carrier plate, with beads therein or thereon, is removed from the wells containing hybridoma cells, and positioned onto a tray. This tray may be filled with a selected buffer solution into which the beads are rinsed by immersion. This immersion step may be repeated two or three times. Subsequent to the rinse, the carrier plate with treated beads will be immersed in another tray preferably containing 96 wells, each containing a solution of a chemical agent that will occupy all the sites on the beads that have not adsorbed antibodies from the hybridoma medium as described above. Examples of the chemical agents which may be utilized are ethanol-di-amine or polyvinylpyrrolydone. After this step, which is not mandatory and depends upon the circumstances which may last between 15 and 45 minutes, the beads are again rinsed in the same fashion as previously described. Thereafter, the carrier plate with the treated beads is positioned onto another tray preferably containing 96 wells. If, for example, the ELISA procedure is to be utilized, the wells of the tray may be filled with an enzyme-labelled-protein A, or preferably enzyme-labelled anti-mouse antibodies. Following incubation and a rinsing procedure, the reacted beads can be placed in a tray which wells are filled with the enzyme substrate linked to a chromogen. After the colour reaction develops, this latter tray's wells can be read in a spectrophotometer, or even visually, in accordance with known ELISA procedures. It is understood that other assay procedures may also utilize the multi-well assembly and components of the present invention.

Thus, the present invention provides a multi-well screening device which is useful in immunoassay procedures and which researchers may use in the simultaneous processing of a number of targets in the detection and quantification of biological substances.

## Claims

1. A carrier plate (26), useful in immunoassay procedures and comprising:
a generally rectangular support body (40),
a plurality of posts (42) arranged in a plurality of rows and extending from a face of the body (40) with each post having a substantially spherical bead (44) at its distal end adapted to bind an immunogenic substance to its surface, each substantially spherical bead (44) being integral with the respective post and not being intended to be removable either from the respective post (42) or generally from the support body (40),
a generally rectangular wall (41) extending from the support body (40) to surround the plurality of posts (42), the wall having two shorter sides (10) and two longer sides (11), and
the wall (41) being squared off at two corners (12, 13) common to a shorter side (10) as an aid to alignment of the carrier plate with a correspondingly configured tray.

2. A plate according to claim 1, wherein the posts are arranged in orthogonal columns and rows.

3. A plate according to claim 1 or 2 which has 96 posts.

4. A plate according to any preceding claim, wherein the substantially spherical beads are made of plastics, preferably styrene acrylonitrile.

5. A plate according to claim 4 which is integrally formed or moulded.

6. A plate according to claim 5, wherein the entire plate is made of styrene acrylonitrile.

7. A multi-well screening assembly comprising:
a carrier plate (26) in accordance with any one of the preceding claims, and
a tray (28) having a plurality of individualized wells (31) arranged in a plurality of rows and a generally rectangular wall (36) extending to surround the plurality of wells (31), the wall (36) having two shorter sides (14) and two longer sides (15), the wall (36) being squared off at two corners (16, 17) common to a shorter side (14) and being dimensioned and configured for uniquely positioning of the carrier plate (26) on the tray (28) with each post extending from the plate to align with a respective one of the wells.

8. An assembly according to claim 7, wherein each row of wells is separated by a fluid barrier thereby forming a plurality of substantially parallel troughs containing the wells.

9. An assembly according to claim 7, wherein at least one row of wells is separated from the remaining rows in the tray.

10. An enzyme-linked immunosorbent assay ("ELISA") for screening of hybridoma cultures, characterized in that an assembly according to any of claims 7 to 9 is employed as part of the equipment for the assay, with the hybridoma cultures having been grown in the wells (31) of the tray (28) and the substantially spherical beads (44) being immersed in the cultures, removed and tested in accordance with the ELISA procedure known *per se*.

## Patentansprüche

1. Für immunologische Testverfahren geeignete Trägerplatte (26) umfassend:
einen im wesentlichen rechteckigen Tragkörper (40),
eine Vielzahl von Stielen (42), die in einer Vielzahl vor Reihen angeordnet sind und sich aus einer Fläche des Körpers (40) erheben, wobei jeder Stiel an seinem distalen Ende ein im wesentlichen kugelförmiges Köpfchen (44) aufweist, das zur Bindung einer immunogenen Substanz an seiner Oberfläche eingerichtet ist, wobei jedes im wesentlichen kugelförmige Köpfchen (44) integral mit dem entsprechenden Stiel verbunden ist und nicht dafür eingerichtet ist, entweder von dem entsprechenden Stiel (42) oder überhaupt von dem Tragkörper (40) entfernt zu werden,
eine im wesentlichen recteckige Wand (41), die sich von dem Tragkörper (40) erhebt und die Vielzahl von Stielen (42) umgibt, wobei die Wand zwei kürzere Seiten (10) und zwei längere Seiten (11) besitzt und
wobei die Wand (41) an zwei Ecken (12, 13) einer kürzeren Seite (10) abgeschrägt ist, um das Ausrichten der Trägerplatte auf eine entsprechend geformte Schale zu erleichtern.

2. Platte nach Anspruch 1, dadurch gekennzeichnet, daß die Stiele in rechtwinkligen Spalten und Reihen angeordnet sind.

3. Platte nach Anspruch 1 oder 2, gekennzeichnet durch 96 Stiele.

4. Platte nach einem der vorhergehenden Ansprüche, dadurch gekennzeichnet, daß die im wesentlichen kugelförmigen Köpfchen aus Kunststoff, vorzugsweise Styrol-Acrylnitril, hergestellt sind.

5. Platte nach Anspruch 4, dadurch gekennzeichnet, daß sie integral geformt oder gegossen ist.

6. Platte nach Anspruch 5, dadurch gekennzeichnet, daß die gesamte Platte aus Styrol-Acrylnitril hergestellt ist.

7. Mehrkammer-Testanordnung, umfassend

eine Trägerplatte (26) gemäß einem der vorhergehenden Ansprüche und

eine Schale (28) mit einer Vielzahl von einzelnen Kammern (32), die in einer Vielzahl von Reihen angeordnet sind, und mit einer im wesentlichen rechteckigen Wand (36), die die Vielzahl von Kammern (32) umgibt, wobei die Wand (36) zwei kürzere Seiten (14) und zwei längere Seiten (15) besitzt und wobei die Wand (36) an zwei Ecken (16, 17) einer kürzeren Seite (14) abgeschrägt ist und so dimensioniert und

geformt ist, daß die Trägerplatte (26) auf der Schale (28) auf eine einzige Weise derart angeordnet werden kann, daß jeder Stiel, der sich von der Platte erhebt, mit einer entsprechenden Kammer fluchtend angeordnet ist.

8. Anordnung nach Anspruch 7, dadurch gekennzeichnet, daß jede Reihe von Kammern voneinander durch eine Flüssigkeitsbarriere getrennt ist, wodurch eine Vielzahl von im wesentlichen parallelen Rinnen gebildet wird, die die Kammern enthalten.

9. Anordnung nach Anspruch 7, dadurch gekennzeichnet, daß mindestens eine Reihe der Kammern von den übrigen Reihen in der Schale getrennt ist.

10. ELISA-Test zur Untersuchung von Hybridoma-Kulturen, dadurch gekennzeichnet, daß eine Anordnung gemäß einem der Ansprüche 7 bis 9 als Teil der Testausstattung verwendet wird, wobei die Hybridoma-Kulturen in den Kammern (32) der Schale (28) gezüchtet worden sind und wobei die im wesentlichen kugelförmigen Köpfchen (44) in die Kulturen eingetaucht, wieder entfernt und gemäß dem an sich bekannten ELISA-Verfahren untersucht werden.

**Revendications**

1. Une plaque porteuse (26) adaptée à la mise en oeuvre des procédés d'essais immunologiques et comportant:

un corps de support (40) sensiblement rectangulaire,

une pluralité de tiges (42) disposées en plusieurs rangées et s'étendant à partir d'une face du corps (40), chaque tige comportant à son extrémité distale, une perle (44) sensiblement sphérique, agencée pour retenir une substance immunogène à sa surface, chaque perle sensiblement sphérique (44) étant en une seule pièce avec la tige correspondante et n'étant pas prévue pour être amovible vis-à-vis de la tige correspondante (42) ni, d'une façon générale, du corps de support (40),

une paroi sensiblement rectangulaire (41) s'étendant à partir du corps de support (40) pour entourer la pluralité de tiges (42), la paroi ayant deux plus petits côtés (10) et deux plus grands côtés (11), et

la paroi (41) étant chanfreinée en deux angles (12, 13) communs à un petit côté (10) pour faciliter l'orientation de la plaque porteuse par rapport à un plateau de forme correspondante.

2. Une plaque selon la revendication 1, dans laquelle les tiges sont disposées en rangées et en colonnes perpendiculaires.

3. Une plaque selon la revendication 1 ou la revendication 2, comportant 96 tiges.

4. Une plaque selon l'une quelconque des revendications précédentes, dans laquelle les perles sensiblement sphériques sont réalisées en matière plastique, de préférence en styrol-acrylonitrile.

5. Une plaque selon la revendication 4, qui est formée ou moulée en une seule pièce.

6. Une plaque selon la revendication 5, dans laquelle toute la plaque est réalisée en styrol-acrylonitrile.

7. Un ensemble d'examen à récipients multiples comportant:

une plaque porteuse (26) selon l'une quelconque des revendications précédentes, et

un plateau (28) présentant une pluralité de cuvettes individualisées (31) disposées en plusieurs rangées et une paroi sensiblement rectangulaire (36) s'étendant de manière à entourer la pluralité de cuvettes (31), la paroi (36) ayant deux plus petits côtés (14) et deux plus grands côtés (15), la paroi (36) étant chanfreinée en deux angles (16, 17) communs à un plus petit côté (14) et étant dimensionnée et conformée pour un positionnement unique de la plaque porteuse (26) sur le plateau (28), chaque tige s'étendant à partir de la plaque dans l'alignement de l'une, correspondante, des cuvettes.

8. Un ensemble selon la revendication 7, dans lequel chaque rangée de cuvettes est séparée par un cloisonnement pour les liquides en formant ainsi plusieurs bacs sensiblement parallèles contenant les cuvettes.

9. Un ensemble selon la revendication 7, dans lequel au moins une rangée de cuvettes est séparée du reste des rangées du plateau.

10. Un essai d'immunosorption lié aux enzymes ("ELISA") pour examiner des cultures d'hybridomes, caractérisé en ce qu'un ensemble selon l'une quelconque des revendications 7 à 9 est utilisé en tant que partie de l'équipement d'essai, les cultures d'hybridomes ayant été amenées à croître dans les cuvettes (31) du plateau (28) et les perles sensiblement sphériques (44) étant immergées dans les cultures, retirées et soumises à des essais conformément au procédé ELISA connu en soi.

FIG.1

FIG.2

FIG.2a